(19) **Europäisches Patentamt**
**European Patent Office**
**Office européen des brevets**

(11) **EP 3 135 332 B1**

(12) **EUROPEAN PATENT SPECIFICATION**

(45) Date of publication and mention
of the grant of the patent:
**05.05.2021 Bulletin 2021/18**

(51) Int Cl.:
***A61M 16/04*** *(2006.01)*      ***A61M 16/00*** *(2006.01)*
***A61M 25/00*** *(2006.01)*

(21) Application number: **15182902.5**

(22) Date of filing: **28.08.2015**

(54) **TRACHEAL CATHETER FOR VENTILATORY SUPPORT OF LUNGS BY CONTINUOUS FLOW OF BREATH GASES DURING NASOTRACHEAL INTUBATION**

LUFTRÖHRENKATHETER ZUR ATEMUNTERSTÜTZUNG DER LUNGEN DURCH KONTINUIERLICHEN FLUSS VON ATEMGASEN WÄHREND DER NASOTRACHEALEN INTUBATION

CATHÉTER TRACHÉAL POUR SUPPORT VENTILATOIRE DES POUMONS PAR ÉCOULEMENT CONTINU DE GAZ DE RESPIRATION LORS DE L'INTUBATION NASOTRACHEAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**01.03.2017 Bulletin 2017/09**

(73) Proprietor: **CHIRANA Medical, a.s.**
**916 01 Stara Tura (SK)**

(72) Inventors:
• **Török, Pavol**
**093 01 Vranov nad Toplou (SK)**

• **Candík, Peter**
**093 03 Vranov nad Toplou (SK)**

(74) Representative: **Porubcan, Róbert**
**Puskinova 19**
**900 28 Ivanka pri Dunaji (SK)**

(56) References cited:
**WO-A1-92/20394**      **WO-A1-2004/067073**
**WO-A2-2013/016094**      **US-A- 3 794 026**
**US-A- 5 188 592**      **US-A1- 2011 130 745**
**US-A2- 2011 004 065**

**Description**

**Field of technology**

The invention concerns a catheter which is inserted into the patient's trachea during a supportive ventilation of the lungs by means of continuous flow of breath gases, which increase the effectiveness of the $CO_2$ washout. The catheter is intended for nasotracheal intubation.

**State of the Art**

A patient during ventilatory support performs part of the ventilatory labor by his or her own respiratory effort and the other part is done by ventilator. It is known in the clinical practice that in order to reach adequate oxygenation in various clinical states - for example, after the denitrogenation of the lungs, it suffices to direct the continual flow of the oxygen to the carina by catheter. Usually, in such cases, the $CO_2$ in the arterial blood rises at pace of 0,2 kPa/min (1,5-2,6 torr/min) - it is so-called apnoeic oxygenation. Apnoeic oxygenation can be realized without the alternating forced flow of the gases in the airways; ventilation of the $CO_2$ is problematic, though. The risks are even higher with the disturbance on the level of alveolar-capillary membrane (ACM) du to increase of Qs/Qt (right-to-left shunt).

According to multiple scientific publications, during problems with high physiological dead space ($V_{DF}$), for example during ARDS, an oxygen is continually applied to the tracheal space (TGI - tracheal gas insufflation), which washes out the anatomic dead space ($V_{DA}$) and thereby lowers the share of the ventilation of the dead space, which lowers the tidal volume of the ventilation. Thanks to the decline in the peak pressure in the airways (Paw max), this subsequently lowers the risk of barotrauma. This method of ventilation with lowering of $V_{DA}$ has significant limitations which consist in the need to achieve a relatively high flow of the gases (for example 4-12 l/min) through thin - for example only 1-2 mm wide - catheter. The pressure needed to drive the gases therefore far surpasses 10 kPa, which is a pressure that destroys the lung parenchyma and thus causes the barotrauma.

Known catheters are usually inserted through the intubation cannula, which undesirably increases the dynamic resistance during exspiratory phase. The flows surpassing 10 l/min cause the positive pressure in trachea on the principle of nozzle - receiving channel; this holds for static and mainly for dynamic state.

These deficiencies limit the development of the abovementioned TGI method. Published patent files such as CN201150702Y, US4091816A, US5339809A, US5389074A, US5507284A do not contribute to the effective solution of the problems concerning the washing out of the dead space. Some solutions such as CN201564930U, CN203677685U, US2005090819A1 use inflatable elements in order to stabilize the position of the catheter in the cavity, but this is not well suited for the respiratory support where a patient exhales outside the catheter. Dual lumen catheters or catheters with other elements increasing its diameter are unsuitable for nasotracheal insertion.

Publication US2006076022A1 describes a nasotracheal catheter for breathing with adjusted distal end which is supposed to help its correct placement. The solution is complicated and it increases the necessary diameter of the catheter which causes problems with its insertion or it requires the increase of the driving pressure.

Publication WO2013/016094 A2 discloses a medical device for the treatment of an airway stenosis, wherein the stenotic region may be in the airway portion selected from the group consisting of larynx, trachea and bronchi. The medical device is an airway dilation and ventilating catheter with an integrated shaft system. The shaft system has a distal shaft portion and a proximal shaft portion and the medical device has a distal end and a proximal end. The distal shaft portion is surrounded by a high pressure balloon located near the ventilating tip. The shaft system contains adjacent dual lumen tubing, including a ventilating lumen permitting passage of oxygen from a ventilation port located near the proximal end of the medical device to facilitate ventilation of the patient and prevent negative pressure pulmonary edema due to attempted breathing during the dilation procedure and the resultant airway blockage. The inner diameter of the ventilation lumen is between about 2 mm and about 4 mm, and is often about 4 mm. The medical device of this document has a ventilating tip with both a forward facing tip opening and radially facing openings to facilitate oxygen flow through the ventilating lumen. The medical device is intended to dilate an airway stenosis and to provide a means to ventilate the airway during the dilation procedure. The medical device is designed to ventilate through the tip opening and four radially facing openings in the ventilating tip, by delivering oxygen via the ventilating lumen for delivery before, during, or after dilation of the airway stenosis. By radially facing openings is intended that the flow through the openings may be at 90 degrees from the flow through the tip opening, but may also be at 30, 45 or 60 degrees or other angles between 0 and 90 degrees. The ventilating tip is located on the distal shaft section, distal to the distal end of the balloon. In one embodiment of the medical device of this document, the inner diameter of the forward facing tip opening is 0.157 inches (4mm) and each of the side openings has an inner diameter of between about 1 and 2 mm and the outer diameter of the integrated shaft system is about 0.236 inches (6 mm).

Published patent file WO92/20394 discloses an apparatus for intratracheal ventilation and intratracheal pulmonary ventilation, where the catheter has lateral openings without the terminal opening.

Publication US 3,794,026 discloses a ventilating apparatus and the catheter has lateral openings between the tracheal ballon and the end of the catheter. The apparatus is designed for full artificial ventilation, not for support in case of spontaneous breathing.

Publication US5188592 (A) discloses a catheter, which comprises an inflation tube for inflating a small balloon for separating the flow of oxygen from the suction. The coude tip comprises a plurality of openings including the end opening and the 2 sidewall openings in the event that one of the end wall opening or the sidewall openings becomes blocked.

Such solution is desired and not known which will reliably and without the need for dangerous increase in the driving pressure ventilate away the $CO_2$ from the lungs, which will function in different modes of flowing of the breath gas and which will not limit patient during movement or common activities.

## Essence of the invention

The abovementioned deficiencies are significantly remedied by the tracheal catheter according to the invention, which is defined in claim 1. The dependent claims 2-14 define preferred embodiments of the invention.

The tubular body of the tracheal catheter has usually, but not exclusively, a circular cross-section and its outer diameter is usually 2 to 8 mm, preferably 3 to 6 mm. The outer diameter of the tubular body ranges from 20% to 50% of the trachea's diameter, preferably 25% to 35% of the trachea's diameter. The diameter of an adult patient's trachea usually ranges from 20 to 25 mm; diameter of a trachea of a child above 10 years is usually at least 8 mm.

The material of the tubular body is at least partially flexible, so it allows non-violent insertion of the catheter into the trachea, whereby it is also solid enough, so the body can be led through the physical cavities during insertion. There exist many tested tubular bodies in the state of the art, for example plastic tube, mainly from polyethylene, which can be used in this invention. It is also part of the prior state of the art that the catheter has a connecting element on one of its ends by which it can be connected to the lung ventilator. By "lung ventilator" we mean any device which is capable of supplying the catheter by breath gas in the prescribed amount and quality; this includes devices which can work on different principles. The connecting element of the catheter can correspond to the output outlet of the lung ventilator; it can be an independent component connected with the end of the tubular body, or the tubular body itself can be shaped into desired shape and size of the connecting element.

The significant feature of the invention is a distal ending of the catheter which includes the ending segment with the terminal opening as defined in claim 1. The terminal opening has a smaller flow cross-section as is the flow cross-section of the tubular body above the ending segment. This tightening of the cross-section produces pneumatic ratios thanks to which the breath gas will flow out of the catheter not only through terminal opening but also through side outlets. By the choice of the ratio between the cross-section of the terminal opening to the inner cross-section of the tubular body and the choice of the ratio between the flow cross-section of the side outlets to the flow cross-section of the terminal opening, the desired ratios of speed and amount of the flow of the breath gas in the ending segment and its outflow to the trachea can be achieved.

According to the invention, the ending segment includes the tightening in the direction of the distal opening as defined in claim 1, which produces the transition from the diameter of the tubular body towards the diameter of the distal opening. This tightening is gradual, without sharp jumps, and it is preferable from the point of view of the easy insertion of the catheter if it is cone shaped. The apex angle of the cone can range from 30° to 90°. The ending segment can be produced as a whole together with the tubular body, for example in such a way that at the end of the plastic tube there is a conical tightening formed; or the ending segment can be an independent component which is safely attached - for example by welding and/or pressing and/or sticking - to the end of the catheter's tubular body.

The existence and direction of the side outlets is important in order to reach the desired effects of the ventilation support. The side outlets may be distributed evenly on the circumference above the terminal opening and they are heading towards the terminal opening, which means that the direction of the outflow of the breath gas from the side outlets forms with the axis of the terminal opening an angle that is less than 90°. This achieves the state where the breath gas flowing out of the side outlets flows towards the trachea's walls not completely perpendicularly, but askew towards the lungs. The even distribution of the side outlets can contain common technological irregularities stemming from production, but it should secure the uniform flowing to the sides of the ending segment in terms of angle and flow. The outflow of the breath gas by the sides secures the position of the distal end of the catheter besides the direct contact with the surface of the trachea, which directs the outflow from the terminal opening approximately to the center of the trachea's cross-section.

The importance of the effect of the outflow of the breath gas through the side, downwards directed outlets is in the fact that the breath gas flows towards the walls, whereby it turbulently rolls on the walls which causes the regulation of the flow in the boundary layer alongside the trachea's walls to the lungs, which effectively washes out the $CO_2$. The washing of the walls does not take place with catheters from the prior state of the art with only single terminal opening, because only main flow comes from the catheter in the direction of the axis of the catheter. Direction of the main flow towards the wall can happen by chance, but such occurrence is always only local and unstable. It can be assumed that it is the flowing of the breath gas by the walls and the pneumatic effects in the boundary layer which contribute significantly to the high effectiveness of the catheter according to this invention. The exact dynamic analysis of the processes of the flowing in the catheter according to this invention is not that important in the end; what is important is that the clinical tests - briefly disclosed after the first and second example of the realization - show that significant positive effects of lung ventilation are achieved.

The side outlets can be produced in different ways. A simple realization from the point of view of production is the one where independent openings are produced in the side walls of the ending segment. In such case at least three opening will be located and distributed in the regular angular gaps on the circumference. The axis of the openings will form an angle less than 90° with the axis of the tubular body; the angle of the conical tightening of the ending segment can contribute to this. Pursuant to the thickness of the wall of the ending segment, the gas flowing out will be directionally led through the route of different length. In order to achieve better directional leading in these openings - especially during the higher speed of the flow -, each wall can be thicker at the places of side outlets than the thickness of the tubular body of the catheter.

From the point of view of the interaction of the flow of the breath gas flowing out of the terminal opening with the turbulent outflows from the side outlets, it is preferable if the side outlets are located above the terminal opening at the distance twice to six times larger than the dimension of the terminal opening.

The side outlets can have different cross-sections; aside from the classical openings with the circular cross-section they can be ellipsoidal or oblong or groove-shaped. In case the ending segment is produced by the pressing in the template, multiple shapes of the side outlets optimized for different groups of patients and disorders can be produced.

The continual flow of the breath gases has to be led to the area above the carina, preferably in the distance of 2,5 to 4 cm above the carina in case of the common adult patient. The catheter is placed in the trachea freely. During high flow of the gases a pneumatic element nozzle - receiving channel is formed, whereby in the receiving channel there is a flow of the gases in the distal direction. This flow helps during the inspirium on one hand - especially in cases of patients where there is at least partial spontaneous breathing effort - and on the other hand it retards eventual exspirium. There is constant positive pressure in cases of non-breathing patients.

The potential energy and size of the pressure created in the receiving channel - with zero flow in the receiving channel (Plmax) - is proportional to the square of the inner diameter of the catheter and the trachea, the size of the driving pressure, which is dependent on the flow and the resistance of the catheter. It holds:

$$Plmax = kin \cdot Pin \cdot (dk / dtr)^2$$

where:

Plmax - pressure produced in the tracheobronchial tree during maximum load; static pressure

kin - flow constant (0,2 - 0,7) depending on the cross-section and flow

Pin - pressure in the insufflation catheter

dk - inner diameter of the catheter (mm)

dtr - inner diameter of the trachea (mm).

Pursuant to the prior state of the art, in case of a partially spontaneously ventilating patient a catheter with a single terminal opening lowers the required inspiratory effort (it helps the inspirium), but on the other hand it produces a pneumatic resistance during exspirium and increases the exspiratory pressure in the distal airways. This creates the modification of EPAP. The size of the PEmax pressure depends on the flow of the gases exspirated during spontaneous ventilation and on the performance of the element nozzle (catheter) - receiving channel (trachea) during constant flow. The pressure (PEmax) during exspirium can in some cases reach the values of several kPa, which is one of the relatively unpleasant phenomena limiting the application of this method according to the prior state of the art. The higher the exspiratory flow of the gases during the spontaneous ventilation is, and the higher the Plmax pressure is, and the higher the flow of the gases through the catheter Qin is, respectively, the higher the PEmax pressure produced during the exspirium is.

During the use of the catheter with side outlets according to this invention a high PEmax or Plmax is not produced, not even during the relatively high flow of the gases through the catheter, which is a much desired effect. The catheter allows significant ventilatory support in cases of at least partially spontaneously ventilating patient with higher flow of the gases and it also significantly eliminates the function of anatomically dead space ($V_{DA}$) by washing it out. The catheter according to this invention eliminates the risk of EPAP compared to other catheters with a single terminal opening according to the prior state of the art.

Thanks to the construction of the catheter, there is a better exchange of the gases during the average flows Qin (10 - 30 l/min), and this effect is achieved thanks to the ventilatory support and washing out of the part of the dead space ($V_{DAX}$). The spontaneous - even if not sufficient - ventilation by the patient is presupposed.

During lower flows Qin (4 - 12 l/min) the washing out of the anatomically dead space by the fresh breath gas takes places, which contributes to the elimination of the $CO_2$ and oxygenation of the patient who is in the borderline respiratory insufficiency; in such case there is a partial elimination of the anatomically dead space ($V_DE$ - dead space elimination). The use of the catheter according to this invention is therefore suitable even in cases of boundary respiratory insufficiency COBD, during terminal disconnection of the patient from the lung ventilator, or in order to lower $V_T$ during borderline low lung pliancy, where Paw increases during the artificial ventilation of the lungs above acceptable values.

The use of the catheter according to this invention for the support of the spontaneous ventilation of the patient in the borderline respiratory insufficiency, where the "physiological reserve" offered by the traditional methods of the exchange of gases in the lungs by the anatomically dead space $V_{DA}$ is used, is also new.

The trachea's space, part of the bronchial bifurcation and nasooral part of the airways constitute approximately 1/2 to 2/3 of the $V_{DA}$ and we call this space in this text an available dead space $V_{DAX}$.

If $V_T$ = 0,55 l and $V_{DA}$ = 2ml/kg in case of the adult man weighing 75 kg, the alveolar ventilation ($V_A$) during the frequency of the ventilation f = 15 d/min is:

$$V_A = ( V_T - V_{DA}) . f. / (0,55 - 0,15 ) . 15 = 6 \text{ l/min}$$

By elimination of $V_{DAX}$ in the volume of 100 ml (2/3 $V_{DA}$, $V_{DA}$ = 0,051) we can achieve following alveolar ventilation: (0,55-0,05)*15 = 7,5 l/min, which is 20% more than when the $V_{DA}$ is left unchanged. The higher the frequency of ventilation is, the better the results are, relatively.

In case of the values in the example, we would achieve lowering of $V_T$ to 0,45 l by the elimination of the $V_{DA}$ with the alveolar ventilation $V_A$=6 l/min left unchanged.

This shows that even with unchanged volume of the spontaneous breathing of the patient it is theoretically possible to increase the alveolar ventilation of the patient by 20% to 35% without the need to increase the ventilation performance - that is, without the need to increase the breathing volume and minute ventilation - if we apply sufficient flow of the gases in order to wash out the dead space to such degree that at the end of the spontaneous exspiration the concentration of $CO_2$ in $V_{DA}$ is 5 to 10 times lower that in the case of spontaneous ventilation without the washing out of the $V_D$. Clinical tests performed on the hundred of patients confirm that the catheter according to this invention has high effectiveness of the $CO_2$ washout.

The flow of the gases through the catheter (Qin) is an important factor for the washing out of the $V_{DA}$ and decrease of the concentration of $CO_2$ in it.

If we want to achieve decrease of the $PCO_2$ in $V_{DA}$ so it is 5 to 10 times lower as compared to spontaneous ventilation in order to eliminate the function of the $V_{DA}$, we have to wash out all available $V_{DA}$ ($V_{DAX}$) during the ending of the exspiration by such a flow that we eliminate the abovementioned approximate 100 ml $V_{DAX}$ which are available for washing out.

The flow of the gases through the catheter (Qin) must be large enough and it will depend on VT, f, Te of spontaneous ventilation and on time constant of the breathing organs (R*C) = $\tau_E$.

The time course of the flow of gases during exspiration is exponentially regressive and the time which has to pass for complete exspiration is Te = > $3\tau$ to 6T.

If $T_E = 6\tau$, then for first $3\tau$ approximately 95% of the volume of the exspirated gases flow out of the lungs, and for the next $3\tau$ only the remaining 5% of the volume.

If we want to decrease the concentration of the $CO_2$ in the $V_{DAX}$ at least 5 times and, of course, increase the concentration of the $O_2$, we have to apply Qin at least in volume which is 1,2 to 2 times the volume of the gases flowing out of the lungs during last $3\tau$ + volume of $V_{DAX}$.

Following calculation of the minimal value of the Qin follows from the abovementioned.

$$Qin = 85 .(V_T . 0.05 + (H * 0,0012 / T_E * 0,5))$$

Qin - flow of the gases through the insufflation catheter (l/min)
$V_T$ - breathing volume of the spontaneous ventilation (l)
H - patients weight in kg

$T_E$ - time of exspiration (sec)

85 - conversion factor to liters/min with constant multiple 1,4 x Qe

For an average patient of 80 kg weight ventilating on the lower limit of the acceptable ventilation $V_T$ = 0,35 l, f = 20 d/min, Te =1,8 sec, the value will be Qin = 10 -14 l/min, se the adequate washing out of the $V_{DAX}$ and the elimination of the function of the part $V_{DA}$ is achieved, which betters the $V_A$ by approximately 5 - 15% without the interference of anything else. The application of approximately double flow of the gases through the catheter will better $V_A$ by 15 - 30%.

It is presumed that during the use of the catheter the breath gas will have the prescribed physical and chemical features which are comparable with physiological values. The gas will have a temperature of 37 $\pm$ 1 °C, it will be saturated by the water vapors (42 $\pm$ 2 mg/liter), that is to 90-100% of relative humidity for the abovementioned temperature. The chemical composition of the breath gas, that is, the concentration of $O_2$, is strictly monitored especially in case of chronic obstructive bronchopulmonary disease, so the ventilation is not damped during better oxygenation. Serious retention of $CO_2$ or exitus in "$CO_2$ narcosis" can be monitored, too. The control and regulation of the temperature of the breath gases will be electronic; it will be monitored during the entering of the gases to the catheter. The monitoring of the concentration of the $O_2$ (FiO_2) will be continuous; it will be monitored by the meter of the concentration of the $O_2$ in gases with the alarm for the upper and lower boundary of the change of concentration.

The invention brings a very simple construction of the catheter for the nasotracheal intubation during ventilatory support of the lungs by a continual flow of the breath gases, where the breath gas flowing out of the catheter effectively washes out the $CO_2$, whereby this does not increase the pressures directed to the peripheral bronchs and to alveolar compartments. The catheter practically eliminates the pressure effects of the flow of the gases in the trachea to the values close to zero and it does not increase inspiratory or exspiratory breathing labor. The risk of barotrauma is low. The stabilization of the catheter in the stable position is simple and reliable and does not hinder the patient in common activity.

## Brief description of drawings

The invention is further disclosed by reference to the drawings 1 to 10. The used scale of the drawings and the ratio of openings and outlets as well as the thickness of the wall of the body do not have to correspond to the description in the drawings and therefore these scales and size ratios cannot be understood as limiting the scope of protection with respect to what is defined in claim 1. The shape of the particular ending segment is for illustration purposes only, too. A person skilled in the art will be able to produce, on the basis of this desription and examples, other realizations of the catheter, which nonetheless fall within the scope of the patent claims.

The drawings show the catheter in the position where the outlet of the terminal opening directs downwards, which corresponds to the common practice of depiction of the human body in the upright position, to which the names of directions "up/down", "above/below" correspond in this document as well. Such names obviously do not denote the absolute definition of the position and in case of different depiction they can be changed accordingly.

Figure 1 depicts a tubular body of the catheter with the connecting element on the one end and with the ending element with tightening on the opposite end. In the lower part of the drawing there is a detail of the ending element.

On figure 2 there is a cross-section of the ending element at the level of its side outlets. Short thick lines depict on this drawing an angular guidance of the cross-section subsequently depicted on the figure 3, where the longitudinal section depicts the skewed guidance of the side outlets. The terminal opening has rounded edges, whereby the decisive dimension is the size of the opening in the smallest cross-section.

Figure 4 depicts the cross-section of the ending segment which is pressed out of the material of the body, whereby it forms a whole with this body. Figure 5 subsequently depicts the realization of the ending segment which is attached to the end of the tubular body.

Figure 6 is a cross-section of the ending segment at the level of side outlets formed by the circumferential groove.

Figure 7 depicts a longitudinal section of the ending segment; the section runs through one of the three joints which connect the upper and lower part of the ending segment.

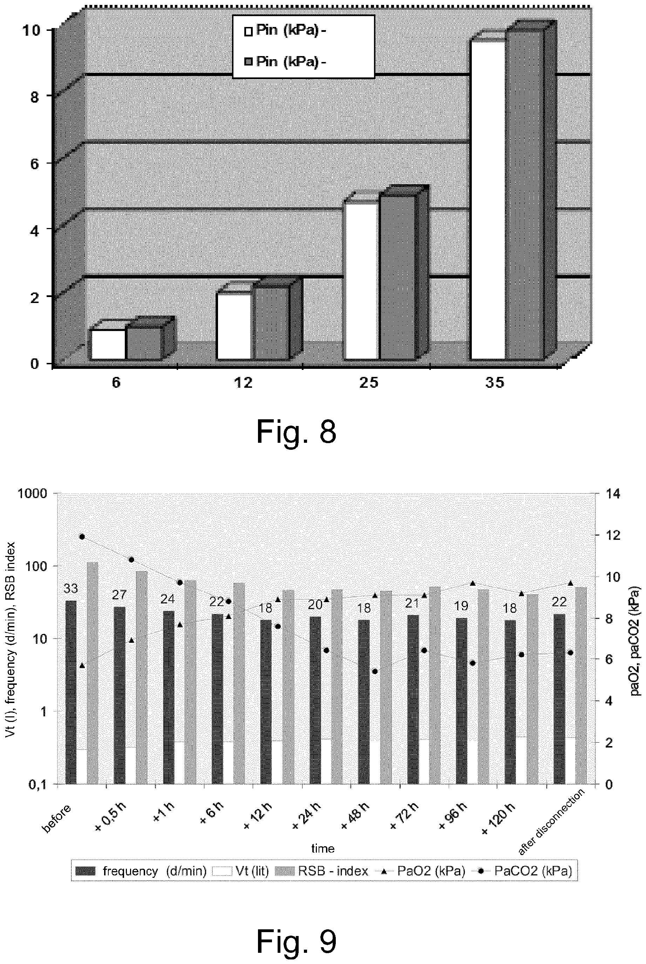

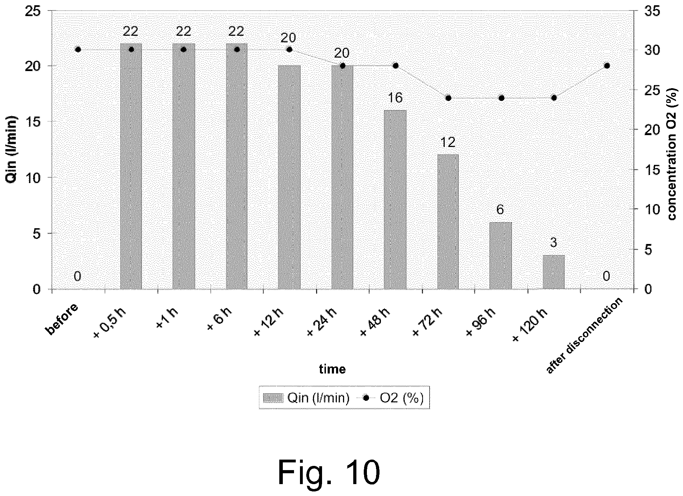

The dependence of the flow of the gases and the driving pressure is depicted in the graph on the figure 8. On the x axis is the flow in l/min, on the y axis is the pressure of the propulsive breath gas in kPa for two realizations of the catheter. Figure 9 is a graph of the clinical application on the set of patients, which depicts in the temporal dependence the respiratory rate and the concentration of PaO_2, PaCO_2. Figure 10 depicts the temporal course of the flow of gases through the catheter and the concentration of FiO_2 during clinical application.

**Examples of realization of the invention**

Example 1

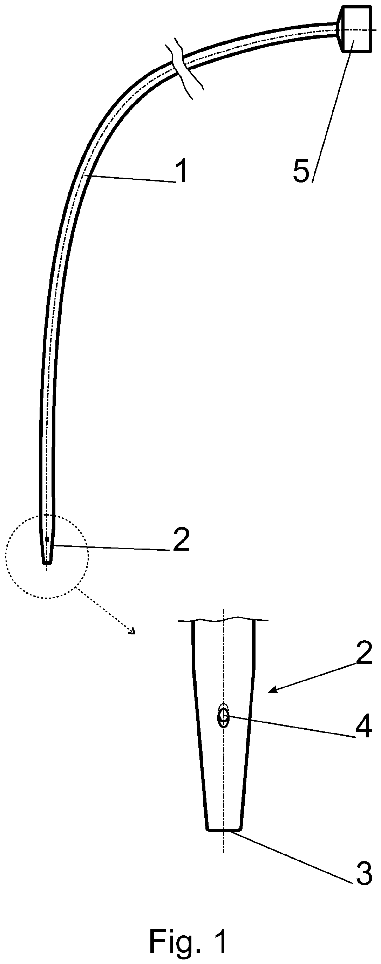

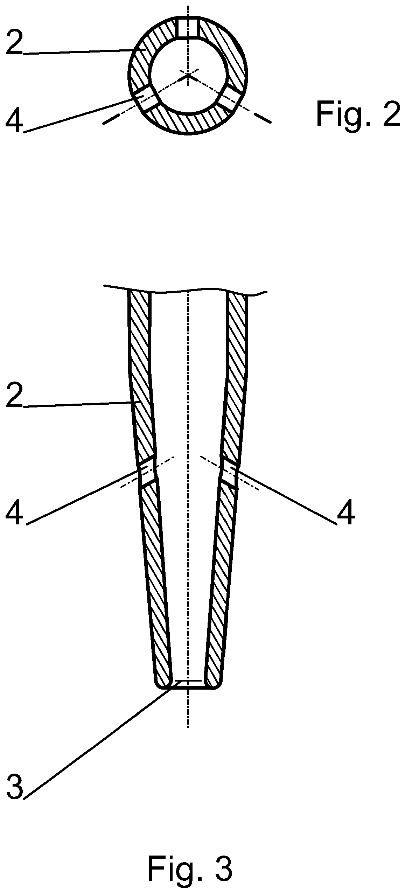

In this example according to figures 1 to 3 and 8 to 10 the catheter is designed for persons of 40-70 kg weight. The catheter has tubular body 1 which is formed by flexible plastic tube with the inner diameter of 3,5 mm and with outer diameter of 4,8 mm. The length of the body 1 is approx. 45 cm.

On the one end of the tubular body 1 there is a connecting element 5 attached which is by its shape and size adjusted for the connection to the output of the particular lung ventilator.

The other end of the plastic tube is formed by the ending element 2 which is tightened in the cone shape and it is ended by the mouth of the terminal opening 3. In this example the ending segment 2 is produced directly from the material of the ending of the body 1 and it forms a single whole with it. The tightening is formed by the gradual transition from the inner diameter of the tube of the body 1 to the diameter of the terminal opening 3. In this zone for tightening, three cylindrical openings are distributed above the terminal opening 3 and these form the side outlets 4. These openings are heading downwards by its outer side, that is, they have the outflow directed towards the terminal opening 3. In this example, these openings are produced by drilling and therefore they have a circular cross-section. Their slope is supported by the conical shape of the ending segment 2.

The side outlets 4 are distributed equally from the point of view of the angle, which means that the three openings these are distributed in 120° around the circumference of the cross-section of the ending segment 2.

Driving pressure Pin necessary for the transport of the desired volume of the gases has been measured, and it is 6, 12, 24 and 34 l/min. Pressure situation in the trachea with the diameter 19 mm has been checked as well. Results are in the figure 8.

For the most common needs of the ventilatory support, with flows which range from 10 to 25 l/min, it is possible to achieve successful ventilation with driving pressure Pin which does not surpass 10 kPa, which is a safe limit from the point of view of the barotrauma.

The results of the clinical tests have shown that the use of the catheter is physiologically very advantageous, with effective oxygenation and washing out of the $CO_2$, whereby the catheter does not cause the undesired pressure changes (PEEP) and does not increase the exspiratory breathing labor even during high flows ranging from 25 to 40 l/min.

Example 2

In this example according to figures 1 to 3 and 8, the ending segment 2 is produced similarly as in example 1. The catheter is intended for the category of the patients who are heavier than 70 kg. It has a body 1 with the inner diameter 4,3 mm and with the outer diameter 5,5 mm.

The static parameters of the system nozzle - receiving channel with trachea with diameter of 20 mm with different flows are depicted on the figure 8.

Catheters described in the examples 1 and 2 were clinically tested. The first group of patients were patients with chronic obstructive pulmonary disease (COPD) with infectious complications; the second group of patients were patients in the phase of disconnection from the long-term artificial lung ventilation, where the previous ventilatory modes of disconnection have been unsuccessful. The patients from the first group usually concerned patients who have overcome the myocardial infarction. The average age of the patients from the first group was 62,5 years, the average weight was 87,1 kg. The average age of the patients from the second group was 64,5 years, the average weight was 96,5 kg. On each of these patients the ventilatory support with the continual flow through the catheters according to this invention has been applied.

Not a single patient from the first group needed intubation and already after 30 minutes after the beginning of the ventilatory support there has been an average decrease of the respiratory rate from $33 \pm 2,8$ to $27 \pm 2,5$ d/min, decrease in $paCO_2$ from $11,9 \pm 1,7$ to $10,8 \pm 1,6$ kPa and increase in $paO_2$ from $5,7 \pm 1,1$ to $6,8 \pm 1,3$ kPa with $FiO_2$ = 0,3. After 24 hours from the beginning of the ventilatory support, the blood gases were adjusted to the values which characterize the partial respiratory insufficiency. The frequency of the spontaneous ventilation diminished to $20 \pm 2,2$, $paCO_2$ decreased to $6,4 \pm 1,2$ kPa and $paO_2$ recorded continual increase reaching the value $8,9 \pm 1,4$ ($FiO_2$ = 0,3) in the 24th hour of the ventilatory support. Ventilatory support lasted for 5 days and then it was ended without complications. In the evolution of the values $PaO_2$ a $PaCO_2$ according to the graph on the figure 9 we can see a high effectiveness of the ventilatory support with the catheter according to this invention. Figure 10 depicts the continual decrease in the flow of the breath gases through the catheter by the end of the ventilatory support.

After extubation and 30 minutes after the beginning of the ventilatory support in the second group of the patients after extubation, the respiratory rate decreased to na $27 \pm 2,5$ d/min, and there has been a continual decrease of the $paCO_2$ to $3,9 \pm 0,9$ in the blood gases as a sign of hyperventilation, which has been probably caused by the continual decrease of $paO_2$ to $8,8 \pm 1,4$ kPa. It was not until 60 minutes after the beginning of the ventilatory support when - with the same respiratory rate - the values of bloods gases increased (paO$_2$ = 9,9 $\pm$ 1,5 kPa, paCO$_2$ = 5,2 $\pm$ 1,1 kPa) and V$_T$ increased as well (0,38 $\pm$ 0,30), which allowed for continuation of the ventilatory support with the continuous flow. After 48 hours the ventilatory support could have been interrupted or ended.

Based on this results it can be stated that the ventilatory support with the continuous flow with the catheter according to this inventions is an effective ventilatory mode and it allows to overcome the phase of coping, for example of infectious complications, without the need for intubation and artificial ventilation of lungs. As a non-invasive ventilatory mode it can be used during disconnection of the patients from the long-term artificial lung ventilation.

The great advantage of this invention is the subjective evaluation of the device by the patients. The patients' assessment of the catheter and coughing up the phlegm was positive; they appreciated the possibility to communicate verbally, receive the food orally, and all this with low need for sedation.

Example 3

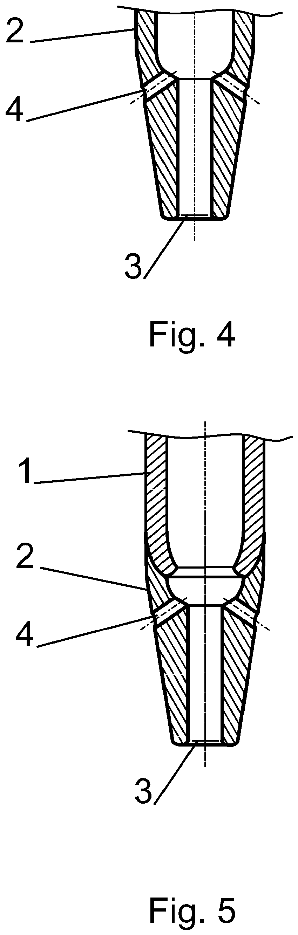

The ending segment 2 is according to the figure 4 created in the multi-part form, where the material at the end of the body 1 is pressed out by heat, and this single act produces the outer tightening with rounded mouth of the terminal opening 3. Inside the ending segment 2 the inner wall does not copy the outer tightening, which means that the wall becomes thicker in the area of side outlets 4. These are circular openings with a fine threaded groove similar to the drilling of the barrels of the firearms, which causes the breath gases flowing out to rotate.

Example 4

The ending segment 2 is and independent plastic component produced independently on the body 1. The ending segment 2 is the welded to the distal end of the body 1 according to figure 5.

The ending segment 2 can be produced by pressing in the template, chip machining, 3D pressing or combination of various technologies. Different ending segments with different geometry of the side outlets and the terminal opening can be separated by different colors.

Example 5

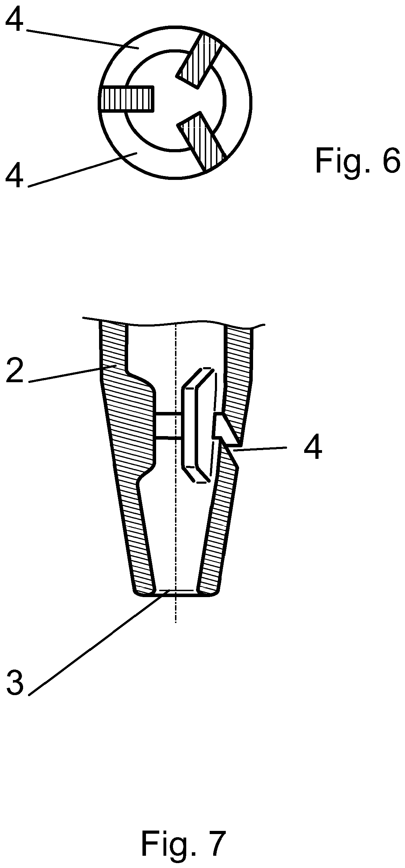

Ending segment 2 according to figures 6 and 7 is a molding from the template. The side outlets are formed by the circumferential groove which is in four places interrupted by the bridges which hold together the upper and lower part of the ending element 2. In order for the bridges to have a sufficient solidity, the joints are produced inside the ending body. The direction of the flow from the side outlets is allowed for by the fact that the groove is skewed and delimited by two cone-shaped cylindrical surfaces.

**Industrial applicability**

The industrial applicability is obvious. According to this invention it is possible industrially and repeatedly produce and use the tracheal catheter for the ventilatory support of the lungs by the continual flow of the breath gases, which increases the effectiveness of the oxygenation as well as significantly eliminates the CO$_2$ from the lungs during given flow of the breath gas.

**List of related symbols**

1- body
2- ending segment
3- terminal opening
4- side outlet
5- connecting element
ARDS - Acute respiratory distress syndrome
EPAP -Expiratory Positive Airway Pressure
COBD - Chronic Obstructive Pulmonary Disease
PEEP -Positive end-expiratory pressure
AKM - alveolar capillary membrane
FiO$_2$ - concentration of the oxygen
TGI - tracheal gas insufflation

**Claims**

1. A tracheal catheter configured for a ventilatory support of lungs by delivery of a continual flow of breath gas via the catheter during a nasotracheal intubation,
   wherein the catheter has a tubular body (1) configured to transport the breath gas in use and made from at least a partially flexible material, which is adjusted for insertion through a nose, throat and glottis to a trachea,
   wherein the catheter has a proximal end and a distal end,
   wherein the catheter has a connecting element (5) configured for connection to a lung ventilator on its proximal end and a terminal opening (3) configured for an outflow of the breath gas to the trachea in a direction of an axis of the body (1) on its distal end,
   wherein the catheter has an ending segment (2), an outlet of which forms the terminal opening (3), the ending segment (2) has side outlets (4) distributed on its circumference and located proximal relative to the terminal opening, the side outlets (4) are configured for outflow of the breath gas therethrough, whereby at least one dimension of the terminal opening (3) is smaller than an inner diameter of the tubular body (1) such that, in use, the breath gas can flow out both through the terminal opening and through the side outlets, and the terminal opening (3) has an area that is smaller than 90% of a flow cross-section of the body (1), the total flow cross-section of the side outlets (4) is at least 10% of the area of the terminal opening (3) and
   wherein the side outlets (4) are leaning towards the terminal opening (3), such that the axis of the side outlets (4) forms an angle less than 90° with the axis of the tubular body (1),
   wherein the ending segment (2) has an outer tightening towards the terminal opening (3),
   wherein the tightening is formed by a transition from a diameter of the tubular body to a diameter of the terminal opening,
   wherein the tightening is gradual and stepless, and
   the side outlets (4) are placed in the tightening.

2. The tracheal catheter according to the claim 1, wherein the outer diameter of the body (1) ranges from 2 to 8 mm.

3. The tracheal catheter according to the claim 1, wherein the outer diameter of the body (1) ranges from 3 to 6 mm.

4. The tracheal catheter according to any of the claims 1 to 3, wherein the tightening is conical with a cone apex angle ranging from 30° to 90°.

5. The tracheal catheter according to any of the claims 1 to 4, wherein the axis of the side outlets (4) forms an angle ranging from 30° to 60° with the axis of the tubular body (1).

6. The tracheal catheter according to any of the claims 1 to 5, wherein the side outlets (4) are equally angularly distributed on the ending segment's (2) circumference.

7. The tracheal catheter according to any of the claims 1 to 6, wherein the side outlets (4) are at least three evenly distributed openings in a wall of the ending segment (2).

8. The tracheal catheter according to any of the claims 1 to 6, wherein the side outlets (4) are formed by a circumferential groove which is interrupted by bridges which hold together an upper part of the ending segment (2) which is located proximal relative to the side outlets and a lower part of the ending segment (2) which is located distal relative to the side outlets.

9. The tracheal catheter according to any of the claims 1 to 8, wherein the side outlets (4) are located proximal relative to the terminal opening (3) at a distance that is twice to six times the at least one dimension of the terminal opening (3).

10. The tracheal catheter according to any of the claims 1 to 9, wherein the side outlets (4) have a circular or ellipsoidal or rectangular outflow cross-section.

11. The tracheal catheter according to any of the claims 1 to 10, wherein the ending segment (2) is produced in one piece with the tubular body (1).

12. The tracheal catheter according to claim 11, wherein the ending segment (2) is produced by means of forming of a material at an end of the body (1).

13. The tracheal catheter according to any of the claims 1 to 12, being configured for flow of the breath gas in the catheter ranging from 4 to 30 l/min.

14. The tracheal catheter according to any of the claims 1 to 13, for support of a spontaneous ventilation of a patient who is bordering on a respiratory insufficiency by his or her spontaneous breathing.

**Patentansprüche**

1. Ein Trachealkatheter konfiguriert zur Atemunterstützung der Lungen durch kontinuierlichen Fluss von Atemgasen durch den Trachealkatheter während der nasotrachealen Intubation,
wobei der Trachealkatheter einen rohrförmigen Körper (1) aufweist, der aus zumindest teilweise flexiblem Werkstoff hergestellt und zum Transport vom Atemgas konfiguriert ist, um das Atemgas durch die Nase, den Larynx, und die Stimmbänder in die Luftröhre einzuführen,
wobei der Trachealkatheter ein proximales Ende und ein distales Ende hat,
und wobei der Trachealkatheter an seinem proximalen Ende ein Anschlusselement (5) zum Anschluss an den Lungenventilator besitzt und an seinem distalen Ende eine Terminalöffnung (3) hat, die zum Austritt des Atemgases in die Luftröhre in Richtung der Körperachse (1) konfiguriert ist,
der Trachealkatheter hat ein Endsegment (2), dessen Mündung die Terminalöffnung (3) bildet, das Endsegment (2) besitzt die Seitenauslässen (4), die an seinem Umfang angeordnet und in der Nähe von der Terminalöffnung (3) platziert sind,
die Seitenauslässen (4) sind für den Abfluss des Atemgases konfiguriert, wobei wenigstens eine Abmessung der Terminalöffnung (3) kleiner als der Innendurchmesser des rohrförmigen Körpers (1) ist, so dass bei Benutzung das Atemgas auch durch die Terminalöffnung (3) als auch durch die seitlichen Seitenauslässen (4) entströmen kann und die Fläche der Terminalöffnung (3) ist kleiner als 90% vom Durchflussquerschnitt des Körpers (1),
die Gesamtquerschnittfläche der Seitenauslässen (4) beträgt wenigstens 10% der Fläche der Terminalöffnung (3) und wobei die Seitenauslässen (4) geneigt in Richtung nach Terminalöffnung (3) stehen und zwar so, dass die Achse der Seitenauslässen (4) einen Winkel bilden, der kleiner als 90° ist,
wobei das Endsegment (2) die äußere Verengung in Richtung nach Terminalöffnung (3) besitzt, wo die Verengung einen Übergang vom Durchmesser des rohrförmigen Körpers (1) zum Durchmesser der Terminalöffnung (3) bildet, wobei die Verengung progressiv und stufenlos ist, und die Seitenauslässen (4) an der Verengung platziert sind.

2. Der Trachealkatheter nach Anspruch 1, wo der Außendurchmesser des Körpers (1) im Bereich von 2 bis 8 mm liegt.

3. Der Trachealkatheter nach Anspruch 1, wo der Außendurchmesser des Körpers (1) im Bereich von 3 bis 6 mm liegt.

4. Der Trachealkatheter nach beliebigem Anspruch 1 bis 3, wo die Verengung kegelförmig mit Spitzenwinkel im Bereich 30° bis 90° ist.

5. Der Trachealkatheter nach beliebigem Anspruch 1 bis 4, wo die Achse der Seitenauslässen (4) mit der Achse des rohrförmigen Körpers (1) einen Winkel im Bereich 30°bis 60° einschließt.

6. Der Trachealkatheter nach beliebigem Anspruch 1 bis 5, wo die Seitenauslässen (4) am Umfang des Endsegments (2) winkelförmig regelmäßig angeordnet sind.

7. Der Trachealkatheter nach beliebigem Anspruch 1 bis 6, wo die Seitenauslässen (4) wenigstens drei gleichmäßig in der Wand des Endsegments (2) verteilte Öffnungen sind.

8. Der Trachealkatheter nach beliebigem Anspruch 1 bis 6, wo die Seitenauslässen (4) durch eine durch Brücken unterbrochene Umfangnut gebildet sind, wobei die Brücken den anliegend an die Seitenauslässen (4) platzierten Oberteil des Endsegments (2), als auch den anliegend an die Seitenauslässen (4) platzierten Unterteil des Endsegments (2) zusammenhalten.

9. Der Trachealkatheter nach beliebigem Anspruch 1 bis 8, wo die Seitenauslässen (4) anliegend an die Terminalöffnung (3) im Abstand, der das Zwei- bis Sechsfache zumindest einer Abmessung der Terminalöffnung (3) macht, platziert sind.

10. Der Trachealkatheter nach beliebigem Anspruch 1 bis 9, wo die Seitenauslässen (4) einen Ausflussdurchschnitt haben, der die Kreis- oder Rechteckform oder elliptische Form besitzt.

11. Der Trachealkatheter nach beliebigem Anspruch 1 bis 10, wo das Endsegment (2) in einem Ganzen mit rohrförmigem Körper (1) gebildet ist.

12. Der Trachealkatheter nach Anspruch 11, wo das Endsegment (2) mit Hilfe der Werkstoffverformung am Ende des rohrförmigen Körpers (1) gebildet ist.

13. Der Trachealkatheter nach beliebigem Anspruch 1 bis 12 ist auf einen Durchfluss vom Atemgas im Bereich von 4 bis 30 l/min eingestellt.

14. Der Trachealkatheter nach beliebigem Anspruch 1 bis 13 zur Unterstützung der spontanen Ventilation eines Patienten, der sich mit seiner Atmung an der Grenze der respiratorischen Insuffizienz befindet.

## Revendications

1. Cathéter trachéal conçu pour une ventilation de secours pulmonaire en apportant un flux continu de gaz respiratoire à travers le cathéter pendant l'intubation nasotrachéale,
   ce cathéter ayant un corps tubulaire (1) configuré pour transporter le gaz respiratoire respectif, en matière au moins partiellement élastique, qui est adapté pour être introduit par le nez, le larynx, les cordes vocales dans la trachée, ce cathéter comportant une extrémité proximale et une extrémité distale
   et présentant à son extrémité proximale un élément de connexion (5) destiné à être connecté à un ventilateur pulmonaire, et à son extrémité distale une ouverture terminale (3) configurée pour évacuer le gaz respiratoire vers la trachée selon l'axe du corps (1),
   ce cathéter comportant un segment d'extrémité (2) dont la sortie forme une ouverture terminale (3), le segment d'extrémité (2) ayant des sorties latérales (4) disposées autour de sa circonférence et situées à proximité de l'ouverture terminale (3),
   lesdites sorties latérales (4) étant configurées pour évacuer le gaz respiratoire, au moins une dimension de l'ouverture terminale (3) étant plus inférieure au diamètre intérieur du corps tubulaire (1) de sorte qu'en utilisant le cathéter, le gaz respiratoire peut également s'écouler tant à travers l'ouverture terminale (3) qu'à travers les sorties latérales (4), et l'ouverture terminale (3) possède une surface qui est inférieure à 90% de la section transversale d'écoulement du corps (1),
   la section transversale totale des sorties latérales (4) représentant au moins 10% de la surface de l'ouverture terminale (3),
   et les sorties latérales (4) étant inclinées vers l'ouverture terminale (3) de sorte que l'axe des sorties latérales (4) forme un angle inférieur à 90° avec l'axe du corps tubulaire (1),
   le segment d'extrémité (2) possède un étranglement extérieur orienté vers l'ouverture terminale (3) où cet étranglement forme une zone de passage du diamètre du corps tubulaire (1) au diamètre de l'ouverture terminale (3), l'étranglement étant graduel et continu et les sorties latérales (4) étant situées dans cet étranglement.

2. Cathéter trachéal selon la revendication 1 dans lequel le diamètre extérieur du corps (1) est compris entre 2 et 8 mm.

3. Cathéter trachéal selon la revendication 1, dans lequel le diamètre extérieur du corps (1) est compris entre 3 et 6 mm.

4. Cathéter trachéal selon l'une quelconque des revendications 1 à 3, dans lequel l'étranglement est conique à un angle au sommet compris entre 30° et 90°.

5. Cathéter trachéal selon l'une quelconque des revendications 1 à 4, dans lequel l'axe des sorties latérales (4) forme un angle avec l'axe du corps tubulaire (1), compris entre 30° et 60°.

6. Cathéter trachéal selon l'une quelconque des revendications 1 à 5, dans lequel les sorties latérales (4) sont uniformément disposées angulairement autour de la circonférence du segment d'extrémité (2).

7. Cathéter trachéal selon l'une quelconque des revendications 1 à 6, dans lequel les sorties latérales (4) sont au moins trois ouvertures uniformément disposées dans la paroi du segment d'extrémité (2).

8. Cathéter trachéal selon l'une quelconque des revendications 1 à 6, dans lequel les sorties latérales (4) sont constituées par une rainure circonférentielle interrompue par des ponts qui supportent ensemble la partie supérieure du segment d'extrémité (2) située en adjacence aux sorties latérales (4), ainsi que la partie inférieure du segment d'extrémité (2) située à l'opposé des sorties latérales (4).

9. Cathéter trachéal selon l'une quelconque des revendications 1 à 8, dans lequel les sorties latérales (4) sont situées en adjacence à l'ouverture terminale (3) à une distance qui est deux à six fois d'au moins d'une des dimensions de l'ouverture terminale (3).

10. Cathéter trachéal selon l'une quelconque des revendications 1 à 9, dans lequel les sorties latérales (4) ont une section transversale de sortie sous forme d'un cercle ou d'une ellipse ou d'un rectangle.

11. Cathéter trachéal selon l'une quelconque des revendications 1 à 10, dans lequel le segment d'extrémité (2) est formé d'un seul tenant avec le corps tubulaire (1).

12. Cathéter trachéal selon la revendication 11, dans lequel le segment d'extrémité (2) est formé par façonnage d'un matériau à l'extrémité du corps tubulaire (1).

13. Le cathéter trachéal selon l'une quelconque des revendications 1 à 12 est conçu pour un débit de gaz respiratoire dans le cathéter compris entre 4 et 30 l/mn.

14. Cathéter trachéal selon l'une quelconque des revendications 1 à 13 servant de support ventilatoire spontanée d'un patient qui est à la limite de l'insuffisance respiratoire par sa respiration.

1

5

2

2

4

3

Fig. 1

Fig. 2

Fig. 3

Fig. 4

Fig. 5

Fig. 6

Fig. 7

Fig. 8

Fig. 9

Fig. 10

**EP 3 135 332 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201150702Y
- US 4091816 A
- US 5339809 A
- US 5389074 A
- US 5507284 A
- CN 201564930 U
- CN 203677685 U
- US 2005090819 A1
- US 2006076022 A1
- WO 2013016094 A2
- WO 9220394 A
- US 3794026 A
- US 5188592 A